(19) Europäisches Patentamt
European Patent Office
Office européen des brevets

(11) **EP 3 326 713 B1**

(12) **FASCICULE DE BREVET EUROPEEN**

(45) Date de publication et mention
de la délivrance du brevet:
**13.05.2020 Bulletin 2020/20**

(21) Numéro de dépôt: **17306379.3**

(22) Date de dépôt: **12.10.2017**

(51) Int Cl.:
*B01J 21/04* (2006.01)    *B01J 23/44* (2006.01)
*B01J 35/00* (2006.01)    *B01J 35/02* (2006.01)
*B01J 35/08* (2006.01)    *B01J 37/02* (2006.01)
*B01J 35/10* (2006.01)    *B01J 37/08* (2006.01)
*B01J 37/10* (2006.01)    *B01J 37/16* (2006.01)
*C07C 5/05* (2006.01)    *C07C 7/163* (2006.01)
*C07C 7/167* (2006.01)    *C10G 70/02* (2006.01)

(54) **CATALYSEUR D'HYDROGENATION SELECTIVE D'UNE COUPE D'HYDROCARBURES C3**

KATALYSATOR FÜR SELEKTIVES HYDRIERVERFAHREN EINER C3-KOHLENWASSERSTOFFFRAKTION

CATALYST FOR SELECTIVE HYDROGENATION OF A C3 HYDROCARBON FRACTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.11.2016 FR 1661622**

(43) Date de publication de la demande:
**30.05.2018 Bulletin 2018/22**

(73) Titulaire: **IFP Energies nouvelles**
**92852 Rueil-Malmaison Cedex (FR)**

(72) Inventeurs:
• **BOUALLEG, Malika**
**69100 VILLEURBANNE (FR)**
• **AVENIER, Priscilla**
**38000 GRENOBLE (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**FR-A1- 2 458 524**     **FR-A1- 2 991 197**
**US-A1- 2005 137 433**

Remarques:
Le dossier contient des informations techniques présentées postérieurement au dépôt de la demande et ne figurant pas dans le présent fascicule.

**Description**

**Domaine technique**

Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants.

L'objet de l'invention est de proposer un catalyseur à performances améliorées ainsi qu'un mode de préparation de ce catalyseur, ce catalyseur possédant de très bonnes performances pour les procédés d'hydrogénation sélective des composés hydrocarbonés insaturés présents dans les coupes d'hydrocarbures C3 de vapocraquage et/ou de craquage catalytique.

**Etat de la technique**

Les catalyseurs d'hydrogénation sélective des coupes C3 de vapocraquage et/ou de craquage catalytique sont généralement à base de palladium, sous forme de petites particules métalliques déposées sur un support qui peut être un oxyde réfractaire. La teneur en palladium et la taille des particules de palladium font partie des critères qui ont une importance sur l'activité et la sélectivité des catalyseurs.

La répartition macroscopique des particules métalliques dans le support constitue également un critère important, principalement dans le cadre de réactions rapides et consécutives telles que les hydrogénations sélectives. Il faut généralement que ces éléments se situent dans une croûte à la périphérie du support afin d'éviter les problèmes de transfert de matière intragranulaire pouvant conduire à des défauts d'activité et une perte de sélectivité. Par exemple, le document US2006/025302 décrit un catalyseur pour l'hydrogénation sélective de l'acétylène et de dioléfines, comprenant du palladium réparti de telle façon que 90% du palladium est introduit dans le catalyseur dans une croûte inférieure à 250 μm.

Par ailleurs, afin d'améliorer la sélectivité des catalyseurs d'hydrogénation sélective, et en particulier des catalyseurs hydrogénation sélective des composés hydrocarbonés insaturés présents dans les coupes d'hydrocarbures C3 de vapocraquage et/ou de craquage catalytique, il a été proposé dans l'art antérieur d'ajouter un second métal choisi parmi le groupe IB, de préférence de l'argent, afin d'obtenir des catalyseurs bimétalliques palladium-Argent (Pd-Ag). De tels catalyseurs sont décrits dans les documents FR 2882531 et FR2991197. L'ajout d'argent dans la phase active du catalyseur a pour effet principal de diminuer la dispersion métallique du palladium, mais ne fait pas par contre changer la répartition en tailles des particules du catalyseur.

La Demanderesse a découvert de manière surprenante que les performances d'un catalyseur de palladium, le palladium étant en partie réparti dans une croûte en périphérie du support, pouvaient être nettement améliorées dans un procédé d'hydrogénation sélective des composés hydrocarbonés insaturés présents dans les coupes d'hydrocarbures C3 de vapocraquage et/ou de craquage catalytique lorsque ledit catalyseur comprend une phase active constituée uniquement de palladium, i.e. qu'il ne comprend pas de métal autre que le palladium dans la phase active, et en particulier de l'argent, ledit catalyseur présentant une dispersion métallique du palladium inférieure à 20%, avec une teneur en palladium et un support poreux de surface spécifique bien déterminés. Un tel catalyseur a pu être obtenu par un procédé de préparation comprenant une étape d'imprégnation colloïdale ainsi qu'une étape de traitement hydrothermal spécifique, engendrant un frittage du catalyseur, ayant pour effet de diminuer la dispersion métallique du palladium au sein du catalyseur, et permettant de manière inattendue, d'obtenir un catalyseur présentant des performances accrues en terme de sélectivité.

En effet, le catalyseur selon l'invention présente une sélectivité importante permettant une hydrogénation des composés acétyléniques et/ou alléniques et/ou dioléfines, tout en limitant l'hydrogénation totale des mono-oléfines (propane dans le cas des coupes C3), et en limitant toute réaction d'oligomérisation ou de polymérisation provoquant une diminution du rendement en propylène et une désactivation précoce du catalyseur.

**Objets de l'invention**

Un premier objet concerne un catalyseur comprenant une phase active constituée uniquement de palladium, et un support poreux d'alumine, dans lequel :

- la teneur en palladium dans le catalyseur est comprise entre 0,0025 et 1% poids par rapport au poids total du catalyseur ;
- au moins 80% poids du palladium est réparti dans une croûte à la périphérie du support poreux, l'épaisseur de ladite croûte étant comprise entre 125 et 225 μm ;
- la surface spécifique du support poreux est comprise entre 70 et 160 m²/g telle que mesurée par physisorption à l'azote ;
- la taille moyenne des particules de palladium est comprise entre 4 et 10 nm ;
- la dispersion métallique D du palladium est inférieure à 20%.

De préférence, la dispersion métallique D du palladium est inférieure ou égale à 18%. Avantageusement, la teneur en palladium dans le catalyseur est comprise entre 0,025 et 0,8% poids par rapport au poids total du catalyseur.
De préférence, la surface spécifique du support poreux est comprise entre 70 et 150 $m^2/g$. Avantageusement, le volume total poreux du support est compris entre 0,1 et 1,5 cm3/g.
De préférence, le support poreux comprend entre 0,0050 et 0,25% en poids de soufre par rapport au poids total du catalyseur.
Le palladium se présente sous la forme de particules de taille moyenne comprise entre 4 à 10 nm.
Un autre objet concerne un procédé de préparation d'un catalyseur selon l'invention comprenant les étapes suivantes :

a) on prépare une suspension colloïdale d'oxyde de palladium ou d'hydroxyde de palladium en phase aqueuse par mélange d'une solution aqueuse (I) comprenant au moins un hydroxyde sélectionné dans le groupe constitué par les hydroxydes d'alcalins et les hydroxydes d'alcalino-terreux et d'une solution aqueuse (II) comprenant au moins un précurseur de palladium;
b) on imprègne ladite solution obtenue à l'étape a) sur un support poreux d'alumine;
c) on soumet le support poreux imprégné obtenu à l'étape b) à une maturation à l'état humide pendant 0,5 à 40 h afin d'obtenir un précurseur de catalyseur ;
d) on sèche le précurseur de catalyseur obtenu à l'étape c) à une température comprise entre 70°C et 200°C pendant une durée comprise entre 0,5 et 20 h ;
e) optionnellement, on calcine le catalyseur séché obtenu à l'étape d) à une température comprise entre 250 et 900°C ;
f) on réalise un traitement hydrothermal du catalyseur séché obtenu à l'étape d) ou du catalyseur calciné obtenu à l'étape e) à une température comprise entre 500°C et 900°C, sous air, comprenant entre 150 et 5000 grammes d'eau par kg d'air ;
g) optionnellement, on soumet le catalyseur obtenu à l'issue de l'étape f) à un traitement réducteur par mise en contact avec un gaz réducteur.

Avantageusement, dans l'étape a), le précurseur du palladium est sélectionné dans le groupe constitué par le chlorure de palladium, le nitrate de palladium et le sulfate de palladium.
Avantageusement, à l'étape f), on réalise un traitement hydrothermal du catalyseur séché obtenu à l'étape d) ou du catalyseur calciné obtenu à l'étape e) à une température comprise entre 600°C et 800°C, sous air, comprenant entre 300 et 4500 grammes d'eau par kg d'air. Un autre objet concerne un procédé d'hydrogénation sélective comprenant la mise en contact d'une coupe C3 de vapocraquage et/ou de craquage catalytique avec le catalyseur selon l'invention ou préparé selon l'invention, dans lequel la température comprise entre 0 et 300°C, à une pression comprise entre 0,1 et 10 MPa, à un ratio molaire hydrogène/(composés polyinsaturés à hydrogéner) entre 0,1 et 10 et à une vitesse volumique horaire V.V.H. comprise entre 0,1 et 100 $h^{-1}$ pour un procédé réalisé en phase liquide, à un ratio molaire hydrogène/(composés polyinsaturés à hydrogéner) entre 0,5 et 1000 et à une vitesse volumique horaire V.V.H. entre 100 et 40000 $h^{-1}$ pour un procédé réalisé en phase gazeuse.

**Description détaillée de l'invention**

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe IB selon la classification CAS correspond aux métaux de la colonne 11 selon la nouvelle classification IUPAC.

1. Définitions

Dispersion métallique des particules (D)

La dispersion de particules est un nombre sans unité, souvent exprimé en %. La dispersion est d'autant plus grande que les particules sont petites. Elle est définie dans publication de R. Van Hardeveld et F. Hartog, «The statistics of surface atoms and surface sites on metal crystals», Surface Science 15, 1969, 189-230.

Définition de l'épaisseur de croûte de palladium

Afin d'analyser la répartition de la phase métallique sur le support, on mesure une épaisseur de croûte par microsonde de Castaing (ou microanalyse par microsonde électronique). L'appareil utilisé est un CAMECA XS100, équipé de quatre cristaux monochromateurs permettant l'analyse simultanée de quatre éléments. La technique d'analyse par microsonde de Castaing consiste en la détection de rayonnement X émis par un solide après excitation de ses éléments par un faisceau d'électrons de hautes énergies. Pour les besoins de cette caractérisation, les grains de catalyseur sont enrobés dans des plots de résine époxy. Ces plots sont polis jusqu'à atteindre la coupe au diamètre des billes ou extrudés puis métallisés par dépôt de carbone en évaporateur métallique. La sonde électronique est balayée le long du diamètre de cinq billes ou extrudés pour obtenir le profil de répartition moyen des éléments constitutifs des solides.

Lorsque le palladium est reparti en croûte, sa concentration locale diminue généralement progressivement lorsqu'elle est mesurée en partant du bord du grain catalytique vers l'intérieur. Une distance du bord du grain, à laquelle la teneur locale en palladium devient nulle, ne peut souvent pas être déterminée avec précision et reproductibilité. Afin de mesurer une épaisseur de croûte qui est significative pour la majorité des particules de palladium, l'épaisseur de croûte est définie comme la distance au bord du grain contenant 80 % en poids de palladium.

Elle est définit dans la publication de L. Sorbier et al. "Measurement of palladium crust thickness on catalyst by EPMA" Materials Science and Engineering 32 (2012). A partir du profil de répartition obtenu par la microsonde de Castaing (c(x)), on peut calculer la quantité cumulée $Q(y)$ de palladium dans le grain en fonction de la distance y au bord du grain de rayon r.

Pour une bille (i.e. pour des grains de catalyseur non conforme à l'invention):

Pour une bille (i.e. pour des grains de catalyseur non conforme à l'invention):

$$Q(y) = \int_{-r}^{-y} c(x) 4\pi.x^2 dx + \int_{y}^{r} c(x) 4\pi.x^2 dx$$

Pour un extrudé :

$$Q(y) = \int_{-r}^{-r+y} c(x) 2\pi.x dx + \int_{r-y}^{r} c(x) 2\pi.x dx$$

avec

    r : rayon du grain ;
    y : distance au bord du grain ;
    x : variable d'intégration (position sur le profil).

On suppose que le profil de concentration suit le diamètre pris de x = - r à x = + r(x = 0 étant le centre).

$Q(r)$ correspond ainsi à la quantité totale de l'élément dans le grain. On résout ensuite numériquement l'équation suivante en y :

$$\frac{Q(y)}{Q(r)} = 0,8$$

c étant une fonction strictement positive, Q est donc une fonction strictement croissante et cette équation possède une seule solution qui est l'épaisseur de croûte.

Définition de l'épaisseur de croûte de palladium

2. Catalyseur

L'invention concerne un catalyseur comprenant une phase active constituée uniquement de palladium, et un support poreux d'alumine, dans lequel :

- la teneur en palladium dans le catalyseur est comprise entre 0,0025 et 1% poids par rapport au poids total du catalyseur, de façon préférée entre 0,025 et 0,8 % poids, et encore plus préférentiellement entre 0,1 et 0,75 % en poids ;
- au moins 80% poids du palladium est réparti dans une croûte à la périphérie du support poreux, l'épaisseur de ladite croûte étant comprise entre 125 et 225 $\mu$m ;
- la surface spécifique du support poreux est comprise entre 70 et 160 m$^2$/g, de manière préférée entre 70 et 150 m$^2$/g, et encore plus préférentiellement entre 80 et 140 m$^2$/g ;
- la dispersion métallique D du palladium est inférieure à 20 %, de préférence inférieure ou égale à 18%.

La taille moyenne des particules de palladium est comprise entre 4 à 10 nm, de préférence entre 3 et 6 nm. La taille moyenne des cristallites est déduite des mesures de dispersion métallique des particules (D), en appliquant les relations dispersion-taille de particules connues de l'homme du métier et décrites dans "Analyse physico-chimique des catalyseurs industriels, Chapitre I, Éditions Technip, Paris 2001".

Le support poreux est de l'alumine. L'alumine peut être présente sous toutes les formes cristallographiques possibles : alpha, delta, téta, chi, gamma, etc., prises seules ou en mélange. De manière préférée le support est choisi parmi l'alumine alpha, delta, téta. De manière très préférée on choisit l'alumine alpha.

La surface spécifique du support poreux est comprise entre 70 et 160 m$^2$/g, de manière préférée entre 70 et 150 m$^2$/g, et encore plus préférentiellement entre 80 et 140 m$^2$/g. La surface spécifique BET est mesurée par physisorption à l'azote. La surface spécifique BET est mesurée par physisorption à l'azote selon la norme ASTM D3663-03 tel que décrit dans Rouquerol F.; Rouquerol J.; Singh K. « Adsorption by Powders & Porous Solids: Principle, methodology and applications », Academic Press, 1999.

Le volume total poreux du support est préférentiellement compris entre 0,1 et 1,5 cm$^3$/g, de préférence compris entre 0,2 et 1,4 cm$^3$/g, et encore plus préférentiellement compris entre 0,25 et 1,3 cm$^3$/g. Le volume poreux total est mesuré par porosimétrie au mercure selon la norme ASTM D4284-92 avec un angle de mouillage de 140°, par exemple au moyen d'un appareil modèle Autopore® III de la marque Microméritics®.

Dans un mode de réalisation selon l'invention, le support du catalyseur d'hydrogénation sélective est purement mésoporeux, i.e. qu'il présente un diamètre de pores compris entre 2 et 50 nm, de préférence entre 5 et 30 nm et de manière encore préférée de 8 à 20 nm. Dans un autre mode de réalisation selon l'invention, le support du catalyseur d'hydrogénation sélective est bimodal, le premier mode étant mésoporeux, i.e. qu'il présente un diamètre de pores compris entre 2 et 50 nm, de préférence entre 5 et 30 nm et de manière encore préférée de 8 à 20 nm, et le second macroporeux, i.e. qu'il présente des pores de diamètre supérieur à 50 nm. Ledit support présente avantageusement un volume poreux des pores ayant un diamètre de pores compris entre 50 et 700 nm inférieur à 20 % du volume poreux total du support, de préférence inférieur à 18 % du volume poreux total du support et de manière particulièrement préférée inférieur à 15 % du volume poreux total du support.

Le support peut comprendre éventuellement du soufre. La teneur en soufre comprise dans le support peut être comprise entre 0,0050 et 0,25% en poids par rapport au poids total du catalyseur, de préférence entre 0,0075 et 0,20% en poids.

Selon l'invention, le support poreux se présente sous forme de billes, de trilobes, d'extrudés, de pastilles, ou d'agglomérats irréguliers et non sphériques dont la forme spécifique peut résulter d'une étape de concassage. De manière très avantageuse, le support se présente sous forme de billes ou d'extrudés. De manière encore plus avantageuse, le support se présente sous forme de billes. Le diamètre des billes est comprise entre 1 mm et 10 mm, de préférence entre 2 et 8 mm, et encore plus préférentiellement entre 2 et 6 mm.

3. Procédé de préparation

L'invention concerne également un procédé de préparation du catalyseur. Le dépôt de la solution de palladium sur le support peut être réalisé selon toutes les techniques connues de l'homme du métier. De préférence, la solution de palladium est déposée par méthode colloïdale.

Plus particulièrement, le procédé de préparation du catalyseur selon l'invention comprend d'une manière générale les étapes suivantes:

a) on prépare une suspension colloïdale d'oxyde de palladium ou d'hydroxyde de palladium en phase aqueuse par mélange d'une solution aqueuse (I) comprenant au moins un hydroxyde sélectionné dans le groupe constitué par les hydroxydes d'alcalins et les hydroxydes d'alcalino-terreux et d'une solution aqueuse (II) comprenant au moins un précurseur de palladium;
b) on imprègne ladite suspension colloïdale obtenue à l'étape a) sur un support poreux comprenant au moins un oxyde réfractaire sélectionné dans le groupe constitué par la silice, l'alumine et la silice-alumine;
c) on soumet le support poreux imprégné obtenu à l'étape b) à une maturation à l'état humide pendant 0,5 et 40 h afin d'obtenir un précurseur de catalyseur ;

d) on sèche le précurseur de catalyseur obtenu à l'étape c) à une température comprise entre 70°C et 200°C pendant une durée comprise entre 0,5 et 20 h ;

e) optionnellement, on calcine le catalyseur séché obtenu à l'étape d) à une température comprise entre 250 et 900°C ;

f) on réalise un traitement hydrothermal du catalyseur séché obtenu à l'étape d) ou du catalyseur calciné obtenu à l'étape e) à une température comprise entre 500°C et 900°C, sous air, comprenant entre 150 et 5000 grammes d'eau par kg d'air ;

g) optionnellement, on soumet le catalyseur calciné obtenu à l'issue de l'étape e) à un traitement réducteur par mise en contact avec un gaz réducteur.

Les différentes étapes sont explicitées en détail ci-après.

a) préparation d'une suspension colloïdale d'oxyde de palladium ou d'hydroxyde de palladium en phase aqueuse

La suspension colloïdale est généralement obtenue par hydrolyse du cation palladium en milieu aqueux, ce qui conduit à la formation de particules d'oxyde ou d'hydroxyde de palladium en suspension.

La solution aqueuse d'hydroxyde d'alcalins ou d'hydroxyde alcalino-terreux est généralement sélectionnée dans le groupe constitué par les solutions aqueuses d'hydroxyde de sodium, les solutions aqueuses d'hydroxyde de magnésium. De manière préférée, de préférence la solution aqueuse est une solution aqueuse d'hydroxyde de sodium.

Le sel précurseur du palladium est généralement sélectionné dans le groupe constitué par le chlorure de palladium, le nitrate de palladium et le sulfate de palladium. De manière très préférée, le sel précurseur du palladium est le nitrate de palladium.

Typiquement, on approvisionne dans un appareillage adapté la solution aqueuse comprenant au moins un sel précurseur du palladium [appelée aussi ici solution (II)] puis la solution aqueuse comprenant au moins un hydroxyde d'alcalins ou d'alcalino-terreux [appelée aussi ici solution (I)]. Alternativement, les solutions (I) et (II) peuvent être versées simultanément dans l'appareillage. De préférence, la solution aqueuse (II) puis la solution aqueuse (I) est versée dans l'appareillage.

La suspension colloïdale reste généralement dans l'appareillage pendant un temps de séjour compris entre 0 et 20 heures.

Les concentrations de la solution (I) et (II) sont généralement choisies afin d'obtenir un pH de la suspension colloïdale compris entre 1,0 et 3,5. Ainsi, le pH de la suspension colloïdale peut être modifié pendant ce temps de séjour par ajout de quantités d'acide ou de base compatibles avec la stabilité de la suspension colloïdale.

En général, la température de préparation est comprise entre 5°C et 40°C et de manière préférée entre 15°C et 35°C.

La concentration en palladium est de préférence comprise entre 5 et 150 millimoles par litre (mmol/L), de manière plus préférée entre 8 et 80 millimoles par litre.

b) dépôt de la suspension colloïdale préparée à l'étape a) par imprégnation sur un support, aluminique

La suspension colloïdale préparée à l'étape 1a) est ensuite imprégnée sur une alumine.

Le support peut éventuellement subir un ensemble de traitements avant l'étape d'imprégnation, tel que des calcinations ou des hydratations.

La suspension colloïdale est de préférence versée sur le support. De préférence, le volume de la suspension colloïdale imprégné sur le support est compris entre 0,9 et 1,1 fois le volume poreux du support. Ce processus peut être réalisé soit de façon discontinue, c'est-à-dire que l'étape de préparation de la suspension colloïdale précède l'étape d'imprégnation sur le support et que l'essentiel de la suspension colloïdale est envoyée en une seule fois vers l'étape d'imprégnation, soit en continu, c'est-à-dire que le produit obtenu dans l'étape a) est envoyé en continu après ajustement du temps de séjour de la suspension colloïdale à l'étape b).

On peut par exemple citer comme processus continu, un processus où les solutions (I) et (II) sont versées simultanément dans un bac qui se déverse en continu dans une zone comprenant le support à imprégner.

c) maturation du support imprégné lors de l'étape b) pendant une durée comprise entre 0,5 et 40 heures

Après imprégnation, le support imprégné est maturé à l'état humide pendant 0,5 à 40 h, de manière préférée pendant 1 à 30 h. Des durées plus longues ne sont pas exclues, mais n'apportent pas nécessairement d'amélioration.

d) séchage du précurseur de catalyseur obtenu à l'étape c)

Le précurseur du catalyseur est séché afin d'éliminer toute ou une partie de l'eau introduite lors de l'imprégnation, à une température comprise entre 70°C et 200°C. La durée du séchage est comprise entre 0,5 h et 20 h. Des durées plus longues ne sont pas exclues, mais n'apportent pas nécessairement d'amélioration.

Le séchage est généralement effectué sous air de combustion d'un hydrocarbure, de préférence du méthane, ou sous air chauffé comprenant entre 0 et 80 grammes d'eau par kilogramme d'air de combustion, un taux d'oxygène compris entre 5% et 25% volume et un taux de dioxyde de carbone compris entre 0% et 10% volume.

e) calcination sous air de combustion du catalyseur séché obtenu à l'étape d) (étape optionnelle)

Après séchage, le catalyseur peut être calciné sous air, de préférence de combustion, et plus préférentiellement un air de combustion du méthane, comprenant entre 40 et 80 gramme d'eau par kg d'air, un taux d'oxygène compris entre 5% et 15% volume et un taux de $CO_2$ compris entre 4% et 10% volume. La température de calcination est généralement comprise entre 250°C et 900°C, de préférence comprise entre environ 300°C et environ 500°C. La durée de calcination est généralement comprise entre 0,5 h et 5 h.

f) traitement hydrothermal du catalyseur séché obtenu à l'étape d) ou calciné obtenu à l'étape e)

Après l'étape d) de séchage ou l'étape e) de calcination, le catalyseur subit un traitement hydrothermal sous air, de préférence sous air de combustion, comprenant entre 150 et 5000 grammes d'eau par kg d'air, de préférence entre 300 et 4500, et encore plus préférentiellement entre 500 et 4000 grammes d'eau par kg d'air.

La température du traitement hydrothermal est comprise entre 500°C et 900°C, de préférence comprise entre 600°C et 800°C.

La durée du traitement hydrothermal est généralement comprise entre 0,5 et 5 heures.

g) réduction de l'oxyde ainsi supporté obtenu à l'étape f), de préférence au moyen d'hydrogène gazeux (étape optionnelle)

Le catalyseur est généralement réduit. Cette étape est de préférence réalisée en présence d'un gaz réducteur, soit in-situ, c'est-à-dire dans le réacteur où est réalisée la transformation catalytique, soit ex-situ. De manière préférée, cette étape est effectuée à une température comprise entre 80°C et 180°C, de manière encore plus préférée entre 100°C et 160°C.

La réduction est réalisée en présence d'un gaz réducteur comprenant entre 25 vol% et 100 vol% d'hydrogène, de préférence 100% volume d'hydrogène. L'hydrogène est éventuellement complété par un gaz inerte pour la réduction, de préférence de l'argon, de l'azote ou du méthane.

La réduction comprend généralement une phase de montée en température puis un palier. La durée du palier de réduction est généralement comprise entre 1 et 10 heures, de préférence entre 2 et 8 heures.

La Vitesse Volumétrique Horaire (V.V.H) est généralement comprise entre 150 et 3000, de préférence entre 300 et 1500 litres de gaz réducteur par heure et par litre de catalyseur.

Utilisation du catalyseur

Le catalyseur selon l'invention peut être utilisé dans les procédés d'hydrogénation sélective des composés hydrocarbonés insaturés présents dans les coupes d'hydrocarbures C3 de vapocraquage et/ou de craquage catalytique. Le procédé d'hydrogénation sélective selon l'invention vise à éliminer lesdits hydrocarbures polyinsaturés présents dans ladite charge à hydrogéner sans hydrogéner les hydrocarbures mono-insaturés. Plus particulièrement, le procédé d'hydrogénation sélective selon l'invention vise à hydrogéner sélectivement le propadiène et le méthylacétylène

Ainsi, par exemple, la coupe C3 de vapocraquage peut avoir la composition moyenne suivante : de l'ordre de 90% poids propylène, de l'ordre de 3 à 8% poids de propadiène et méthylacétylène, le reste étant essentiellement du propane.

Dans certaines coupes C3, entre 0,1 et 2% poids de C2 et de C4 peut aussi être présent. Les spécifications concernant les concentrations de ces composés polyinsaturés pour les unités de pétrochimie et de polymérisation sont très basses : 20-30 ppm poids de MAPD (méthylacétylène et propadiène) et propylène supérieure à 95% pour le propylène qualité chimique et moins de 10 ppm poids voire jusqu'à 1 ppm poids pour la qualité « polymérisation » et propylène supérieure à 99%.

La mise en œuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré.

La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en œuvre technologique du procédé d'hydrogénation sélective selon l'invention peut également être avantageusement réalisée par l'implantation d'au moins dudit catalyseur supporté dans une colonne de distillation réactive ou dans des réacteurs - échangeurs. Le flux d'hydrogène peut être introduit en même temps que la charge à hydrogéner et/ou en un ou plusieurs points différents du réacteur.

L'hydrogénation sélective des coupes C3 peut être réalisée en phase gazeuse ou en phase liquide, de préférence en phase liquide. Une réaction en phase liquide permet d'abaisser le coût énergétique et d'augmenter la durée de cycle du catalyseur.

D'une manière générale, l'hydrogénation sélective des coupes C3 s'effectue à une température comprise entre 0 et 300°C, de préférence entre 20 et 100°C, à une pression comprise entre 0,1 et 10 MPa, de préférence entre 0,5 et 5 MPa, à un ratio molaire hydrogène/(composés polyinsaturés à hydrogéner) entre 0,1 et 10 et à une vitesse volumique horaire V.V.H. (définie comme le rapport du débit volumique de charge sur le volume du catalyseur) comprise entre 0,1 et 100 h$^{-1}$ pour un procédé réalisé en phase liquide, à un ratio molaire hydrogène/(composés polyinsaturés à hydrogéner) entre 0,5 et 1000 et à une vitesse volumique horaire V.V.H. entre 100 et 40000 h$^{-1}$ pour un procédé réalisé en phase gazeuse.

Exemples

Les exemples présentés ci-dessous visent à démontrer l'amélioration de l'activité catalytique en hydrogénation sélective des catalyseurs selon l'invention. Les exemples 1 à 4 concernent des procédés de préparation de catalyseurs non conformes à l'invention (catalyseurs C1 à C4) et les exemples 5 et 6 concernent des procédés de préparation d'un catalyseur selon l'invention (catalyseurs C5 et C6).

L'exemple 7 concerne la mesure de la dispersion métallique D des catalyseurs C1 à C6. L'exemple 8 concerne l'application de ces catalyseurs dans une réaction d'hydrogénation sélective d'une coupe C3.

Ces exemples sont présentés à titre d'illustration et ne limitent en aucune manière la portée de l'invention.

Exemple 1 : préparation d'un catalyseur C1 non conforme

Une suspension colloïdale d'oxyde de Pd est préparée sous agitation à 25°C par dilution de 1,8 g d'une solution de nitrate de palladium Pd(NO$_3$)$_2$ contenant 8,5 % poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80 g d'une alumine dont la surface spécifique est de 71 m$^2$/g mise en forme sous forme de billes. Une étape de maturation du support imprégné avant séchage d'une durée de 3 h est effectuée sous air en milieu confiné et humide. Le solide obtenu est séché sous air pendant 20 h à 120°C.

Le catalyseur C1 obtenu est séché sous air à 120°C, puis est calciné pendant 2 heures à 450°C sous un flux d'air de combustion avec une VVH de 500 litres d'air de combustion par litre de catalyseur et par heure. L'air de combustion contient environ 60 g d'eau par kg d'air sec.

Le catalyseur C1 ainsi préparé comprend 0,19% en poids de palladium par rapport au poids total de catalyseur.

La caractérisation du catalyseur C1 par microsonde de Castaing montre que 80% du Pd est réparti sur une croûte d'épaisseur environ 222 μm.

La dispersion métallique D du palladium du catalyseur C1 est de 26%.

Exemple 2 : préparation d'un catalyseur C2 non conforme

Une suspension colloïdale d'oxyde de Pd est préparée sous agitation à 25°C par dilution de 1,8 g d'une solution de nitrate de palladium Pd(NO$_3$)$_2$ contenant 8,5 % poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80 g d'une alumine dont la surface spécifique est de 71 m$^2$/g mise en forme sous forme de billes. Une étape de maturation du support imprégné avant séchage d'une durée de 3 h est effectuée sous air en milieu confiné et humide. Le solide obtenu est séché sous air pendant 20 h à 120°C.

Le catalyseur C2 obtenu est séché sous air à 120°C, puis est calciné pendant 2 heures à 650°C sous un flux d'air de combustion avec une VVH de 4000 litres d'air de combustion par litre de catalyseur et par heure. L'air de combustion contient environ 60 g d'eau par kg d'air sec.

Le catalyseur C2 ainsi préparé comprend 1,5% en poids de palladium par rapport au poids total de catalyseur.

La caractérisation du catalyseur C2 par microsonde de Castaing montre que 80% du Pd est réparti sur une croûte d'épaisseur environ 500 $\mu$m.

La dispersion métallique D du palladium du catalyseur C2 est de 25%.

Exemple 3 : préparation d'un catalyseur C3 non conforme (méthode par imprégnation à sec)

Une solution aqueuse de nitrate de palladium est préparée à 25°C par dilution de 3,5 g d'une solution de nitrate de palladium contenant 8,5 % poids de palladium, avec de l'eau déminéralisée à un volume qui correspond au volume poreux du support alumine.

Cette solution est ensuite imprégnée (par la méthode d'imprégnation à sec) sur 100 gramme d'une alumine dont la $S_{BET}$ est de 300 m$^2$/g. Cette alumine est sous forme de billes dont le diamètre moyen est de 3 mm.

Une étape de maturation du support imprégné avant séchage d'une durée de 20 h est effectuée sous air en milieu confiné et humide. Le solide obtenu est séché sous air pendant 2 h à 120°C. Le catalyseur 1 obtenu est séché sous air à 120°C, puis est calciné pendant 2 heures à 650°C sous un flux d'air de combustion avec une VVH de 3000 litres d'air de combustion par litre de catalyseur et par heure. L'air de combustion contient environ 4000 g d'eau par kg d'air sec.

Le catalyseur C3 ainsi préparé comprend 0,19% en poids de palladium par rapport au poids total de catalyseur.

La caractérisation du catalyseur C3 par microsonde de Castaing montre que 80% du Pd est réparti sur une croûte d'épaisseur environ 500 $\mu$m.

La dispersion métallique D du palladium du catalyseur C3 est de 30%.

Exemple 4 : préparation d'un catalyseur C4 non conforme

Une suspension colloïdale d'oxyde de Pd est préparée sous agitation à 25°C par dilution de 1,8 g d'une solution de nitrate de palladium Pd(NO$_3$)$_2$ contenant 8,5 % poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80 g d'une alumine dont la surface spécifique est de 300 m$^2$/g mise en forme sous forme de billes. Une étape de maturation du support imprégné avant séchage d'une durée de 3 h est effectuée sous air en milieu confiné et humide.

Le catalyseur 4 obtenu est séché sous air à 120°C, puis est calciné pendant 2 heures à 650°C sous un flux d'air de combustion avec une VVH de 500 litres d'air de combustion par litre de catalyseur et par heure. L'air de combustion contient environ 4000 g d'eau par kg d'air.

Le catalyseur C4 ainsi préparé comprend 0,18% en poids de palladium par rapport au poids total de catalyseur.

La caractérisation du catalyseur C4 par microsonde de Castaing montre que 80% du Pd est réparti sur une croûte d'épaisseur environ 210 $\mu$m.

La dispersion métallique D du palladium du catalyseur C4 est de 23%.

Exemple 5 : préparation d'un catalyseur C5 conforme

Une suspension colloïdale d'oxyde de Pd est préparée sous agitation à 25°C par dilution de 1,8 g d'une solution de nitrate de palladium Pd(NO$_3$)$_2$ contenant 8,5 % poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80 g d'une alumine dont la surface spécifique est de 90 m$^2$/g mise en forme sous forme de billes. Une étape de maturation du support imprégné avant séchage d'une durée de 3 h est effectuée sous air en milieu confiné et humide.

Le catalyseur C5 obtenu est séché sous air à 120°C, puis est calciné pendant 2 heures à 650°C sous un flux d'air de combustion avec une VVH de 500 litres d'air de combustion par litre de catalyseur et par heure. L'air de combustion contient environ 3500 g d'eau par kg d'air.

Le catalyseur C5 ainsi préparé comprend 0,18% en poids de palladium par rapport au poids total de catalyseur.

La caractérisation du catalyseur C5 par microsonde de Castaing montre que 80% du Pd est réparti sur une croûte d'épaisseur environ 195 $\mu$m.

La dispersion métallique D du palladium du catalyseur C5 est de 14%.

Exemple 6 : préparation d'un catalyseur C6 conforme

Une suspension colloïdale d'oxyde de Pd est préparée sous agitation à 25°C par dilution de 1,8 g d'une solution de nitrate de palladium Pd(NO₃)₂ contenant 8,5 % poids de palladium Pd avec environ 45 ml d'eau déminéralisée, puis ajout d'environ 10 ml d'une solution de soude pour arriver à un pH de 2,4. La suspension est ensuite diluée avec de l'eau déminéralisée à un volume qui correspond au volume poreux du support alumine. Cette solution est ensuite imprégnée sur 80 g d'une alumine dont la surface spécifique est de 150 m²/g mise en forme sous forme de billes. Une étape de maturation du support imprégné avant séchage d'une durée de 3 h est effectuée sous air en milieu confiné et humide.

Le catalyseur 6 obtenu est séché sous air à 120°C, puis est calciné pendant 2 heures à 650°C sous un flux d'air de combustion avec une VVH de 500 litres d'air de combustion par litre de catalyseur et par heure. L'air de combustion contient environ 3500 g d'eau par kg d'air.

Le catalyseur C6 ainsi préparé comprend 0,30 % en poids de palladium par rapport au poids total de catalyseur.

La caractérisation du catalyseur C6 par microsonde de Castaing montre que 80% du Pd est réparti sur une croûte d'épaisseur environ 150 μm.

La dispersion apparente du palladium du catalyseur C6 est de 12%.

Exemple 7 : Mesure de dispersion métallique D des catalyseurs C1 à C6

Les mesures de dispersions métalliques sont effectuées par chimisorption de monoxyde de carbone CO, sur le catalyseur préalablement réduit sous 1 litre d'hydrogène par heure et par gramme de catalyseur, avec une montée en température de 300°C/h et un palier de deux heures à 150°C. Le catalyseur est ensuite balayé pendant 1 heure à 150°C sous hélium puis refroidi jusqu'à 25°C sous hélium.

La chimisorption de CO est réalisée à 25°C en dynamique selon les pratiques habituelles connues de l'homme du métier, elle conduit à un volume de CO chimisorbé, à partir duquel l'homme du métier peut calculer le nombre de molécules de CO chimisorbés. Un rapport stoechiométrique d'une molécule de CO par atome de Pd de surface est pris en compte pour calculer le nombre d'atomes de Pd de surface. La dispersion est exprimée en % d'atomes de Pd de surface par rapport à la totalité des atomes de Pd présent dans l'échantillon du catalyseur. La dispersion métallique D du palladium des catalyseurs C1 à C6 est présentée dans le tableau 1 ci-après.

Tableau 1: Dispersion métallique D des catalyseurs C1 à C6

| | $S_{BET}$ support (m²/g) | Teneur en Pd (%pds) | Méthode d'imprégnation | Traitement hydrothermal | Dispersion (%) | Taille de la croute (μm) |
|---|---|---|---|---|---|---|
| C1 (non conforme) | 71 | 0,19 | colloïdale | 450°C/60g d'eau* | 26 | 222 |
| C2 (non conforme) | 71 | 1,5 | colloïdale | 650°C/60g d'eau* | 25 | 500 |
| C3 (non conforme) | 300 | 0,19 | Imprégnation a sec | 650°C/4000g d'eau* | 30 | 500 |
| C4 (non conforme) | 300 | 0,18 | colloïdale | 650°C/4000g d'eau* | 23 | 210 |
| C5 (conforme) | 90 | 0,18 | colloïdale | 650°C/3500g d'eau* | 14 | 195 |
| C6 (conforme) | 150 | 0,30 | colloïdale | 650°C/3500g d'eau* | 12 | 150 |
| *par kg d'air sec | | | | | | |

Exemple 8 : utilisation des catalyseurs C1 à C6 pour l'hydrogénation sélective de la coupe C3 vapocraquage

Une charge comprenant 92,47% poids de propylène, 4,12%poids de propane, 1,18%poids de méthylacétylène (MA), 1,63% poids de propadiène (PD) est traitée avec les catalyseurs C1 à C7. Avant réaction les catalyseurs d'hydrogénation sélective sont actifs sous un flux d'hydrogène à 160°C pendant 2h.

25 ml de catalyseur sont places dans un réacteur tubulaire en mode up flow. La pression est maintenue à 30 bars (3 MPa) et la température à 27°C. Une vitesse horaire volumique (V.V.H.) de 50h⁻¹ est appliquée. Le rapport molaire H₂/MAPD varie entre 0, 5, 10 mol/mol. La composition de la charge et de l'effluent est mesuré en continue en sortie du réacteur par chromatographie en phase gaz. Les oligomères gazeux sont définis comme les oligomères non piégé par les différents filtres de l'unité et détecté par la colonne chromatographie (composé jusqu'à 6 carbones). Les performances des catalyseurs C1 à C6 sont exprimées dans le tableau 2 ci-après.

Tableau 2: sélectivités mesurées en hydrogénation sélective de la coupe C3

| Catalyseur | Sélectivité en propylène pour une conversion de 90 % du MAPD* (%) | Sélectivité en oligomères gazeux pour une conversion de 90 % du MAPD* (%) |
|---|---|---|
| C1 (non conforme) | 52 | 13 |
| C2 (non conforme) | 55 | 14 |
| C3 (non conforme) | 62 | 18 |
| C4 (non conforme) | 60 | 14 |
| C5 (conforme) | 64 | 6 |
| C6 (conforme) | 71 | 4 |
| *MAPD = méthylacétylène et propadiène | | |

Le catalyseur C5 et C6 sont conformes à l'invention. Ils présentent à la fois une très bonne sélectivité en propylène et en production d'oligomères gazeux. Les catalyseurs les plus sélectifs sont ceux qui ont une croute de palladium fine, i.e. inférieure à 225 microns ainsi qu'une dispersion inférieure à 20%.

Ces catalyseurs sont préparés sur des supports présentant une surface spécifique supérieure à 70 m$^2$/g et inférieure à 160 m$^2$/g car au-delà, l'acidité du support catalyse la réaction d'oligomérisation, tel qu'illustré dans l'exemple 4 pour lequel le catalyseur C4 engendre une quantité importante d'oligomères, conduisant à un cokage précoce du catalyseur et une durée de vie plus courte de ce dernier. Des surfaces spécifiques trop élevées et/ou la teneur en palladium trop forte ainsi que des dispersions en CO trop élevées conduisent aux catalyseurs C1 à C4 peu performants, produisant trop de propane, composé non désiré pour cette application, et des teneurs en oligomères gazeux symptomatiques d'un production d'oligomères plus lourds et de coke trop importante et donc nuisibles à la durée de vie du catalyseur et à la bonne conduite de l'unité de d'hydrogénation.

**Revendications**

1. Catalyseur comprenant une phase active constituée uniquement de palladium, et un support poreux d'alumine, dans lequel :

    - la teneur en palladium dans le catalyseur est comprise entre 0,0025 et 1% poids par rapport au poids total du catalyseur ;
    - au moins 80% poids du palladium est réparti dans une croûte à la périphérie du support poreux, l'épaisseur de ladite croûte étant comprise entre 125 et 225 μm telle que mesurée par microsonde de Castaing ;
    - la dispersion métallique D du palladium est inférieure à 20% ;
    - la surface spécifique du support poreux est comprise entre 70 et 160 m$^2$/g telle que mesurée par physisorption à l'azote ;
    - la taille moyenne des particules de palladium est comprise entre 4 à 10 nm telle que déduite des mesures de dispersion métallique des particules.

2. Catalyseur selon la revendication 1, dans lequel la dispersion métallique D du palladium est inférieure ou égale à 18%.

3. Catalyseur selon les revendications 1 ou 2, dans lequel la teneur en palladium dans le catalyseur est comprise entre 0,025 et 0,8% poids par rapport au poids total du catalyseur.

4. Catalyseur selon l'une quelconque des revendications précédentes, **caractérisé en ce que** la surface spécifique du support poreux est comprise entre 70 et 150 m$^2$/g.

**5.** Catalyseur selon l'une quelconque des revendications précédentes, **caractérisé en ce que** le volume total poreux du support est compris entre 0,1 et 1,5 cm$^3$/g tel que mesuré par porosimétrie au mercure.

**6.** Catalyseur selon l'une quelconque des revendications précédentes, **caractérisé en ce que** le support poreux comprend entre 0,0050 et 0,25% en poids de soufre par rapport au poids total du catalyseur.

**7.** Procédé de préparation d'un catalyseur selon l'une quelconque des revendications 1 à 6 comprenant les étapes suivantes :

a) on prépare une suspension colloïdale d'oxyde de palladium ou d'hydroxyde de palladium en phase aqueuse par mélange d'une solution aqueuse (I) comprenant au moins un hydroxyde sélectionné dans le groupe constitué par les hydroxydes d'alcalins et les hydroxydes d'alcalino-terreux et d'une solution aqueuse (II) comprenant au moins un précurseur de palladium;
b) on imprègne ladite solution obtenue à l'étape a) sur un support poreux d'alumine;
c) on soumet le support poreux imprégné obtenu à l'étape b) à une maturation à l'état humide pendant 0,5 à 40 h afin d'obtenir un précurseur de catalyseur ;
d) on sèche le précurseur de catalyseur obtenu à l'étape c) à une température comprise entre 70°C et 200°C pendant une durée comprise entre 0,5 h et 20 h ;
f) on réalise un traitement hydrothermal du catalyseur séché obtenu à l'étape d) à une température comprise entre 500°C et 900°C, sous air, comprenant entre 150 et 5000 grammes d'eau par kg d'air.

**8.** Procédé de préparation selon la revendication 7 dans lequel dans l'étape a), le précurseur du palladium est sélectionné dans le groupe constitué par le chlorure de palladium, le nitrate de palladium et le sulfate de palladium.

**9.** Procédé selon l'une quelconque des revendications 7 ou 8, dans lequel à l'étape f) on réalise un traitement hydrothermal du catalyseur séché obtenu à l'étape d) à une température comprise entre 600°C et 800°C, sous air, comprenant entre 300 et 4500 grammes d'eau par kg d'air.

**10.** Procédé d'hydrogénation sélective comprenant la mise en contact d'une coupe C3 de vapocraquage et/ou de craquage catalytique avec le catalyseur selon l'une quelconque des revendications 1 à 6 ou préparé selon l'une quelconque des revendications 7 à 9, dans lequel la température comprise entre 0 et 300°C, à une pression comprise entre 0,1 et 10 MPa, à un ratio molaire hydrogène/(composés polyinsaturés à hydrogéner) entre 0,1 et 10 et à une vitesse volumique horaire V.V.H. comprise entre 0,1 et 100 h$^{-1}$ pour un procédé réalisé en phase liquide, à un ratio molaire hydrogène/(composés polyinsaturés à hydrogéner) entre 0,5 et 1000 et à une vitesse volumique horaire V.V.H. entre 100 et 40000 h$^{-1}$ pour un procédé réalisé en phase gazeuse.


**Patentansprüche**

**1.** Katalysator, der eine aktive Phase, welche ausschließlich aus Palladium besteht, sowie einen porösen Aluminiumoxidträger umfasst, wobei:

- der Gehalt an Palladium im Katalysator im Bereich von 0,0025 bis 1 Gewichts-% liegt, bezogen auf das Gesamtgewicht des Katalysators;
- mindestens 80 Gewichts-% des Palladiums in einer Kruste im Randbereich des porösen Trägers verteilt ist, wobei die Dicke der Kruste im Bereich von 125 bis 225 $\mu$m gemäß einer Messung mittels Elektronenstrahlmikroanalyse liegt;
- das Palladium eine Dispersion in Metallform D von weniger als 20 % erfährt;
- die spezifische Oberfläche des porösen Trägers gemäß einer Messung mittels physikalischer Stickstoffsorption im Bereich von 70 bis 160 m$^2$/g liegt;
- die mittlere Größe der Palladiumpartikel im Bereich von 4 bis 10 nm liegt, wenn sie von den Messungen der Dispersion in Metallform der Partikel abgeleitet wird.

**2.** Katalysator nach Anspruch 1, wobei das Palladium eine Dispersion in Metallform D von höchstens 18 % erfährt.

**3.** Katalysator nach den Ansprüchen 1 oder 2, wobei der Gehalt an Palladium im Katalysator im Bereich von 0,025 bis 0,8 Gewichts-% liegt, bezogen auf das Gesamtgewicht des Katalysators.

4. Katalysator nach einem beliebigen der vorhergehenden Ansprüche, **dadurch gekennzeichnet, dass** die spezifische Oberfläche des porösen Trägers im Bereich von 70 bis 150 m$^2$/g liegt.

5. Katalysator nach einem beliebigen der vorhergehenden Ansprüche, **dadurch gekennzeichnet, dass** das Gesamtporenvolumen des Trägers gemäß einer Messung mittels Quecksilberporosimetrie im Bereich von 0,1 bis 1,5 cm$^3$/g liegt.

6. Katalysator nach einem beliebigen der vorhergehenden Ansprüche, **dadurch gekennzeichnet, dass** der poröse Träger zwischen 0,0050 und 0,25 Gewichts-% an Schwefel umfasst, bezogen auf das Gesamtgewicht des Katalysators.

7. Verfahren zur Herstellung eines Katalysators nach einem beliebigen der Ansprüche 1 bis 6, die folgenden Schritte umfassend:

   a) Herstellen einer kolloidalen Lösung von Palladiumoxid oder von Palladiumhydroxid in wässriger Phase, indem eine wässrige Lösung (I), die mindestens ein Hydroxid umfasst, welches aus der Gruppe ausgewählt ist, die aus den Alkalimetallhydroxiden und den Erdalkalimetallhydroxiden besteht, mit einer wässrigen Lösung (II) vermischt wird, die mindestens eine Palladiumvorstufe umfasst;
   b) Durchtränken eines porösen Aluminiumoxidträgers mit der Lösung, wie sie in Schritt a) erhalten wurde;
   c) Einwirkenlassen einer Reifung in feuchtem Zustand auf den durchtränkten porösen Träger, wie er Schritt b) erhalten wurde, über einen Zeitraum von 0,5 bis 40 Std., um eine Katalysatorvorstufe zu erhalten;
   d) Trocknen der Katalysatorvorstufe, wie sie in Schritt c) erhalten wurde, bei einer Temperatur zwischen 70 °C und 200 °C über einen Zeitraum im Bereich von 0,5 Std. bis 20 Std;
   f) Durchführen einer hydrothermalen Behandlung des getrockneten Katalysators, wie er in Schritt d) erhalten wurde, bei einer Temperatur im Bereich von 500 °C bis 900 °C, unter Zufuhr von Luft, die 150 bis 5.000 g Wasser pro kg Luft umfasst.

8. Herstellungsverfahren nach Anspruch 7, wobei im Schritt a) die Vorstufe des Palladiums aus der Gruppe ausgewählt ist, die aus Palladiumchlorid, Palladiumnitrat und Palladiumsulfat besteht.

9. Verfahren nach einem beliebigen der Ansprüche 7 oder 8, wobei im Schritt f) eine hydrothermale Behandlung des getrockneten Katalysators, wie er in Schritt d) erhalten wurde, bei einer Temperatur im Bereich von 600 °C bis 800 °C unter Zufuhr von Luft durchgeführt wird, die 300 bis 4.500 g Wasser pro kg Luft umfasst.

10. Selektives Hydrierungsverfahren, im Rahmen dessen eine C3-Fraktion aus einem Dampfcrackvorgang und/oder aus einem katalytischen Crackvorgang mit dem Katalysator in Kontakt gebracht wird, welcher einem beliebigen der Ansprüche 1 bis 6 entspricht und nach einem beliebigen der Ansprüche 7 bis 9 hergestellt ist, wobei die Temperatur im Bereich von 0 bis 300 °C liegt, bei einem Druck im Bereich von 0,1 bis 10 MPa, bei einem Molverhältnis von Wasserstoff / (zu hydrierenden mehrfach ungesättigten Verbindungen) zwischen 0,1 und 10 sowie bei einem stundenbezogenen Volumendurchsatz SVD im Bereich von 0,1 bis 100 h$^{-1}$ für ein Verfahren, das in flüssiger Phase durchgeführt wird, bei einem Molverhältnis von Wasserstoff / (zu hydrierenden mehrfach ungesättigten Verbindungen) zwischen 0,5 und 1.000 sowie bei einem stundenbezogenen Volumendurchsatz SVD im Bereich von 100 bis 40.000 h$^{-1}$ für ein Verfahren, das in der Gasphase durchgeführt wird.

**Claims**

1. A catalyst comprising an active phase constituted solely by palladium, and a porous alumina support, in which:

   • the palladium content in the catalyst is in the range of 0.0025% to 1% by weight with respect to the total weight of the catalyst;
   • at least 80% by weight of the palladium is distributed in a crust at the periphery of the porous support, the thickness of said crust being in the range of 125 to 225 $\mu$m as measured using a Castaing microprobe;
   • the metallic dispersion D of the palladium is less than 20%;
   • the specific surface area of the porous support is in the range of 70 to 160 m$^2$/g as measured by nitrogen physisorption;
   • the average size of the palladium particles is in the range of 4 to 10 nm as deduced from metallic dispersion measurements of the particles.

**2.** The catalyst as claimed in claim 1, in which the metallic dispersion D of the palladium is 18% or less.

**3.** The catalyst as claimed in claim 1 or claim 2, in which the palladium content in the catalyst is in the range of 0.025% to 0.8% by weight with respect to the total weight of the catalyst.

**4.** The catalyst as claimed in any one of the preceding claims, **characterized in that** the specific surface area of the porous support is in the range of 70 to 150 $m^2$/g.

**5.** The catalyst as claimed in any one of the preceding claims, **characterized in that** the total pore volume of the support is in the range of 0.1 to 1.5 $cm^3$/g as measured by mercury porosimetry.

**6.** The catalyst as claimed in any one of the preceding claims, **characterized in that** the porous support comprises in the range of 0.0050% to 0.25% by weight of sulfur with respect to the total weight of the catalyst.

**7.** A process for the preparation of a catalyst as claimed in any one of claims 1 to 6, comprising the following steps:

a) preparing a colloidal suspension of palladium oxide or palladium hydroxide in an aqueous phase by mixing an aqueous solution (I) comprising at least one hydroxide selected from the group constituted by alkali hydroxides and alkaline-earth hydroxides and an aqueous solution (II) comprising at least one precursor of palladium;
b) impregnating said solution obtained in step a) on a porous alumina support;
c) maturing the impregnated porous support obtained in step b) in the wet state for 0.5 to 40 h in order to obtain a catalyst precursor;
d) drying the catalyst precursor obtained in step c) at a temperature in the range of 70°C to 200°C for a duration in the range of 0.5 h to 20 h;
f) carrying out a hydrothermal treatment of the dried catalyst obtained in step d) at a temperature in the range of 500°C to 900°C, in air, comprising in the range of 150 to 5000 grams of water per kg of air.

**8.** The preparation process as claimed in claim 7, in which in step a), the palladium precursor is selected from the group constituted by palladium chloride, palladium nitrate and palladium sulfate.

**9.** The process as claimed in claim 7 or claim 8, in which in step f), a hydrothermal treatment of the dried catalyst obtained in step d) is carried out at a temperature in the range of 600°C to 800°C, in air, comprising in the range of 300 to 4500 grams of water per kg of air.

**10.** A process for selective hydrogenation, comprising bringing a C3 cut from steam cracking and/or catalytic cracking into contact with the catalyst as claimed in any one of claims 1 to 6 or prepared as claimed in any one of claims 7 to 9, in which the temperature is in the range of 0°C to 300°C, at a pressure in the range of 0.1 to 10 MPa, with a molar ratio of hydrogen/ (polyunsaturated compounds to be hydrogenated) in the range of 0.1 to 10 and at an hourly space velocity, HSV, in the range of 0.1 to 100 $h^{-1}$ for a process carried out in the liquid phase, with a molar ratio of hydrogen/(polyunsaturated compounds to be hydrogenated) in the range of 0.5 to 1000 and at an hourly space velocity, HSV, in the range of 100 to 40000 $h^{-1}$ for a process carried out in the gas phase.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2006025302 A
- FR 2882531
- FR 2991197

**Littérature non-brevet citée dans la description**

- **D.R. LIDE.** CRC Handbook of Chemistry and Physics. 2000
- **R. VAN HARDEVELD ; F. HARTOG.** The statistics of surface atoms and surface sites on metal crystals. *Surface Science,* 1969, vol. 15, 189-230
- **L. SORBIER et al.** Measurement of palladium crust thickness on catalyst by EPMA. *Materials Science and Engineering,* 2012, vol. 32
- Analyse physico-chimique des catalyseurs industriels. 2001
- **ROUQUEROL F. ; ROUQUEROL J. ; SINGH K.** Adsorption by Powders & Porous Solids: Principle, methodology and applications. Academic Press, 1999